# EUROPÄISCHE PATENTANMELDUNG

(11) **EP 0 761 816 A2**
(43) Veröffentlichungstag der Anmeldung: **12.03.1997**
(21) Anmeldenummer: 96250180.5
(22) Anmeldetag: 23.08.1996
(51) Int. Cl.: C12N 15/63, C12N 15/82

(54) **Verfahren zur Erhöhung der pflanzlichen Samenproduktion und zur Beschleunigung des Pflanzenwachstums durch zusätzliche plastidäre Pyruvat Phosphat Dikinase**

(30) Priorität: 30.08.1995 DE 19531783
(71) Anmelder: Sheriff, Ahmed, Dr., 12305 Berlin (DE)
(72) Erfinder: Sheriff, Ahmed, Dr., 12305 Berlin (DE)
(74) Vertreter: Miehe, Manfred, Dipl.-Chem.

(57) **Zusammenfassung**

Aus den Daten lassen sich folgende Schlüsse ziehen: Mit Hilfe zusätzlicher plastidärer PPDK können Pflanzen zu schnellerem Wachstum veranlaßt werden. Diese Pflanzen produzieren mehr Samen als der Wildtyp. Die erhöhte Samenproduktion kommt durch schwerere Samenkapseln und höhere Samenmengen pro Samenkapsel zustande. Der Grund für diese Effekte konnte nicht eindeutig geklärt werden, liegt aber mit großer Wahrscheinlichkeit an der Refixierung von Kohlendioxid (siehe Abbildung 1), welches während der Atmung entsteht, da auch nicht-photosynthetisch aktive Gewebe wie Samen vom zusätzlichen Phospho*enol*pyruvat profitieren. Dies führt zu effizienterer Nutzung der zur Verfügung stehenden Kohlenstoffgerüste. Die zusätzliche plastidäre PPDK stellt das für die Refixierung durch PEPCase benötigte Substrat Phospho*enol*pyruvat in größeren Mengen zur Verfügung und behebt so einen zu Energieverschwendung und Kohlenstoffverlust führenden Engpaß. Auch die geringste gemessene Aktivitätserhöhung von 125% führt zu deutlich erhöhter Samenproduktionseffizienz und schnellerem Wachstum der Pflanzen (Abbildung 6, 7 und 8).

Durch die hier beschriebene gentechnische Veränderung mit einer plastidären PPDK sollte sich nahezu jede Pflanze in oben beschriebener Weise verbessern lassen. Die Gentechnik erlaubt zusätzlich, solche Veränderungen sehr schnell herbeizuführen, während die Züchtung eines solchen Merkmals zumindest ein sehr zeitaufwendiger Prozeß ist.

## Beschreibung

### Problemstellung

Während des zwanzigsten Jahrhunderts wurden durch Pflanzenzüchtung und -selektion bemerkenswerte Verbesserungen im Ertrag und der Qualität von Getreidesamen erzielt (Borlaug und Dowswell 1988). Doch solche Zuwächse können nicht fortwährend erzielt werden. Von den vielen schwerwiegenden Problemen mit denen der Mensch konfrontiert ist, ist das Bevölkerungswachstum das größte. Bei der momentanen Geschwindigkeit des Bevölkerungswachstums wird sich die Weltbevölkerung bis zum Jahre 2030 auf 11 Milliarden verdoppelt haben. Das führt schon jetzt zum Verlust von landwirtschaftlich genutzten Gebieten. Um die Weltbevölkerung auch in 40 Jahren noch mit Nahrung zu versorgen, wird es all unserer Erfindungsgabe bedürfen. Die dringende Notwendigkeit, die Nahrungsproduktion zu erhöhen, kann vielleicht unter Zuhilfenahme der Pflanzenmolekularbiologie befriedigt werden. Diese erlaubt den direkten Zugang zu einem immensen Genpool, da sie den Transfer von nützlichen Genen aus jedwedem Organismus in Pflanzen erlaubt.

Pflanzen sind ganz erstaunliche Organismen. Sie können ihren gesamten Energiebedarf mit Hilfe des Sonnenlichtes decken. Zusätzlich generieren sie bei dem, als Photosynthese bekannten Prozeß, Kohlenhydrate aus Kohlendioxid. Diese Stoffe können sowohl als Energiespeicher, zur Biosynthese als auch als strukturelle Bestandteile verwendet werden. Aber nicht alle pflanzlichen Gewebe sind zur Photosynthese befähigt. Zu den nicht-photosynthetisch aktiven Geweben gehören alle, die nicht mit Licht in Kontakt kommen oder aber andere Aufgaben haben, wie beispielsweise die unterirdischen Wurzeln, die Leitgewebe und die reifenden Samen. Im Dunkeln kann natürlich auch keine Photosynthese stattfinden. Die photosynthetisch aktiven Gewebe müssen also während der Lichtphasen genügend Energie produzieren und speichern, um den gesamten Organismus zu versorgen und wenn möglich das Wachstum und die Reproduktion voranzutreiben. Normalerweise erfolgen kurzfristige Speicherung (Stärke, Saccharose) und Transfer (Saccharose) von Energie in Form von Zuckern. Hauptabnehmer von Saccharose sind heranwachsende Blätter und die nicht-photosynthetisch aktiven Pflanzenteile. Die nicht-photosynthetisch aktiven Pflanzenteile müssen auch ihre gesamten organischen Bestandteile aus den transferierten Kohlenstoffgerüsten herstellen (Dennis und Turpin 1990; Mohr und Schopfer 1978; Strasburger *et al.* 1991; Taiz und Zeiger 1991).

Bei der Veratmung der aus der Saccharose stammenden Hexosen Glucose und Fructose können bis zu 38 ATP* gewonnen werden (Voet und Voet 1992). Gleichzeitig wird aber auch das während der Photosynthese fixierte Kohlendioxid wieder freigesetzt. Die Photosynthese benötigt 18 ATP und 12 NADPH für die Produktion einer Hexose (Mohr und Schopfer 1978; Strasburger *et al.* 1991; Taiz und Zeiger 1991). Dies entspricht einer Energiemenge von 54 ATP. Eine einfache Rechnung zeigt, daß von Photosynthese zu Atmung 16 ATP Verlust zu verzeichnen sind.
* Adenosintriphophat (ATP) ist die Energiewährung der Zellen. Die Änderung der freien Energie (ΔG) für die ATP-Hydrolyse beträgt unter physiologischen Bedingungen -50 bis -65 kJ/mol. Ein NADH kann zu 3 ATP umgewandelt werden.

Kann die Atmung den Ernteertrag verringern? Die Antwort lautet: Ja. Eine 20% Verringerung der Atmung kann zu 10 - 20% Steigerung des Ernteertrages führen (Lambers 1985; Wilson und Jones 1982). Nun kann man auf dem Acker schlecht für längere Lichtperioden, optimale Wachstumstemperaturen, optimale Luft- oder Bodenfeuchte sorgen. Deshalb müssen andere Lösungen zur Reduktion des Kohlendioxidverlustes durch Atmung gefunden werden.

### Lösungsansatz

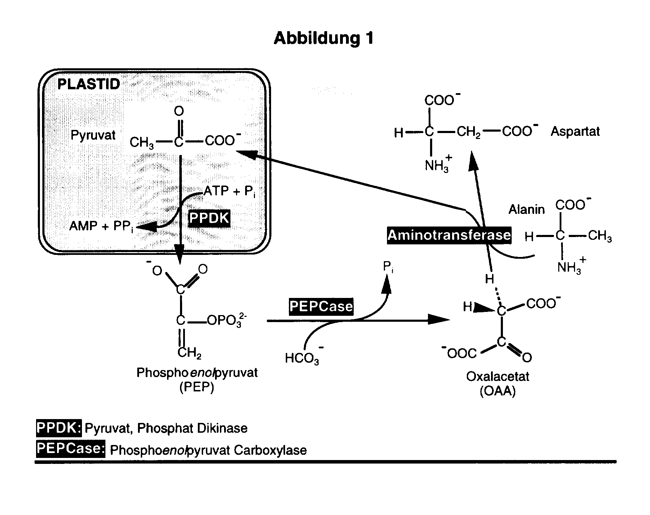

Wie kann das während der Atmung freigesetzte Kohlendioxid unabhängig von der Photosynthese erneut fixiert werden? In Abbildung 1 ist eine biochemische Möglichkeit zur Refixierung von Kohlendioxid aufgeführt (Latzko und Kelly 1983). Die Fixierung wird hier von der Phosphoenolpyruvat Carboxylase (PEPCase; EC 4.1.1.31) vorgenommen. Die PEPCase ist ein Schlüsselenzym im anaplerotischen Stoffwechsel (Auffüllen des Tricarbonsäurezyklus). Die pflanzliche Pyruvat, Phosphat Dikinase (PPDK; EC 2.7.9.1) ist ein plastidäres Enzym von dem vermutet wird, daß es in nicht-photosynthetisch aktiven Geweben Phosphoenolpyruvat (PEP) für die PEPCase-Reaktion zur Verfügung stellt (Latzko und Kelly 1983; Meyer *et al.* 1978).

Das Produkt der Phosphoenolpyruvat Carboxylierung ist Oxalacetat. Dieses kann in einer Transaminasereaktion zu der Aminosäure Asparaginsäure umgewandelt werden. Alanin dient hier zumeist als Substrat. Aus dem Alanin entsteht dabei Pyruvat, welches wieder von der PPDK genutzt werden kann. Durch den in Abbildung 1 gezeigten Zyklus kann Kohlendioxid auf Kosten von zwei energiereichen Phosphatgruppen (oder umgerechnet zwei ATP) fixiert werden. Der Calvin-Zyklus benötigt drei ATP und zwei NADPH+H+ (oder umgerechnet neun ATP) für die Fixierung von Kohlendioxid (Taiz und Zeiger 1991). Die Refixierung von Kohlendioxid durch PPDK und PEPCase kann Pflanzen also helfen, viel Energie zu sparen (sieben ATP pro refixiertem Kohlendioxid) und bewahrt der Pflanze vor allem gleichzeitig einen Teil des bei der Atmung freigesetzten Kohlendioxids.

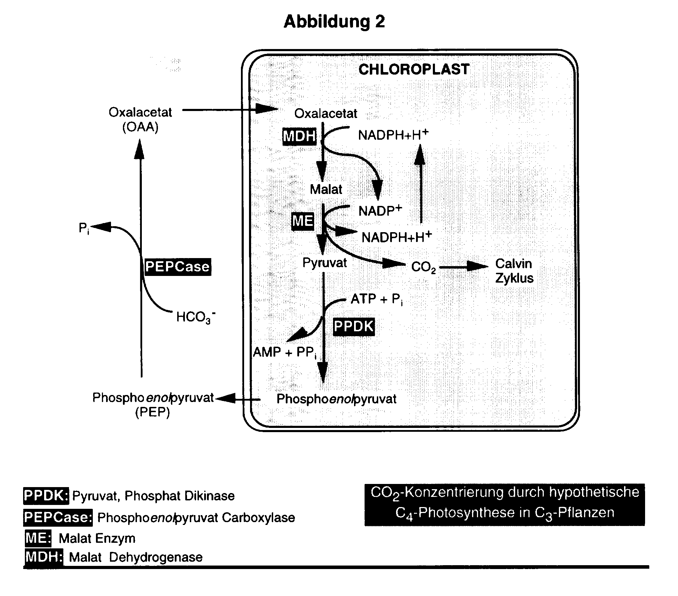

Ein anderer Grund für das Vorkommen von PEPCase und PPDK in photosynthetisch aktiven Geweben von C₃-Pflanzen wird von Aoyagi und Bassham (1986) vorgeschlagen (Abbildung 2). Hierbei dienen PEPCase und PPDK der intrazellulären Kohlendioxidanreicherung, was zu vermehrter Kohlendioxidfixierung durch die Ribulose-1,5-bisphosphat Carboxylase (RubisCo) führte. Diese Art der primären Kohlendioxidfixierung und -anreicherung wird von CAM- und C₄-Pflanzen ausgenutzt. Allerdings gibt es bei CAM Pflanzen eine zeitliche Trennung zwischen PEPCase- (Nacht) und RubisCo-Reaktion (Tag), während C₄-Pflanzen PEPCase (Mesophyllzellen) und RubisCo (Bündelscheidenzellen) räumlich trennen, denn beide Enzyme stehen natürlich auch in Konkurrenz um Kohlendioxid (Dennis und Turpin 1990; Mohr und Schopfer 1978; Strasburger *et al.* 1991; Taiz und Zeiger 1991; Ting 1985).

Latzko und Kelly (1983) schlagen noch zehn weitere biochemische Gründe für das Vorkommen von PEPCase vor. Diese weiteren Einsatzmöglichkeiten ergeben allerdings keine unmittelbaren energetischen Vorteile. Ein Substrat der PEPCase ist Phosphoenolpyruvat. Dieses kann unter anderem aus Pyruvat durch die PPDK generiert werden, so daß die PEPCase eventuell auch durch den Nachschub an Phosphoenolpyruvat limitiert ist. Die enzymatischen Charakteristika und metabolischen Funktionen der PPDK aus nicht-photosynthetisch aktiven Geweben oder C₃-Pflanzen werden allerdings noch nicht endgültig verstanden (Fißlthaler *et al.* 1995; Rosche *et al.* 1994).

Meine Idee besteht nun darin, einen eventuellen Mangel an Phosphoenolpyruvat zu beheben. Meine Hauptannahme ist, daß die PPDK den limitierenden Faktor darstellt. Bringt man Pflanzen nun dazu, größere PPDK-Mengen zu produzieren, müßte sich ein solcher Engpaß beheben lassen. Um diese These zu beweisen, wurde die PPDK-cDNA aus der fakultativen CAM-Pflanze *Mesembryanthemum crystallinum* in *Nicotiana tabacum* (Samsun SNN) eingebracht und überexprimiert. Das Testsystem Tabak dient hier nur als Modell und wurde aufgrund der einfachen Handhabung gewählt. Die PPDK aus M. *crystallinum* dient als Stellvertreter dieser Enzymklasse und wurde aufgrund ihrer Verfügbarkeit gewählt.

### Lösungen

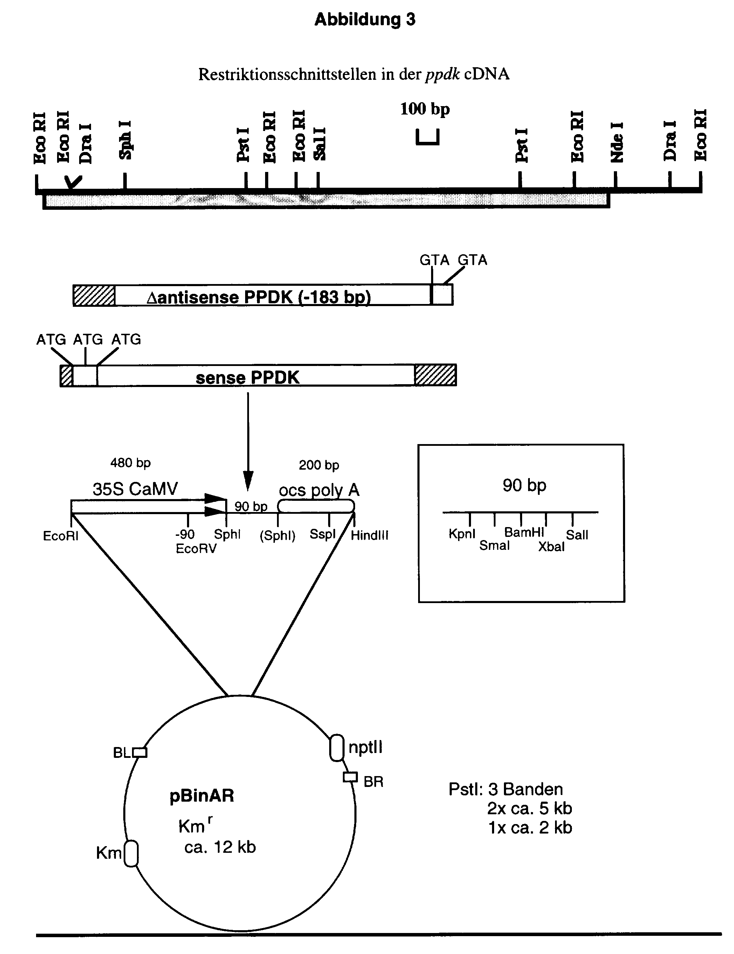

Die cDNA der M. *crystallinum* PPDK kodiert für ein plastidäres Enzym von 103 kDa mit und 94 kDa ohne Plastidenerkennungssequenz (Fißlthaler 1993; Fißlthaler *et al.* 1995). Diese cDNA wurde in Sense-Orientierung in einen binären Expressionsvektor (Bevan 1984) kloniert (siehe Abbildung 3). In einem Kontrollkonstrukt wurde die cDNA in umgekehrter Orientierung (ΔAntisense) in den binären Expressionsvektor kloniert. Dieser umgedrehten cDNA fehlen 183 Basenpaare am ehemaligen 5'-Ende (ΔPPDK). Die *ppdk-*cDNA befand sich in beiden Fällen unter der Kontrolle des konstitutiv, in allen Geweben exprimierten CaMV 35S-Promotors. Eine weitere Kontrolle stellt der Vektor ohne PPDK dar. Dieser Vektor enthielt zur Transformationskontrolle ein β-Glucuronidase-Gen (Vancanneyt *et al.* 1990). Tabak erhielt durch diese binären Vektoren (siehe Abbildung 3) mit Hilfe von *Agrobacterium tumefaciens* zusätzliche Erbinformation. Die T-DNA des binären Plasmids wird von *A. tumefaciens* in Pflanzengenome integriert. Die Integration der T-DNA in das Pflanzengenom erfolgt an einen unbestimmten Ort des Genoms, so daß man bei verschiedenen Transformanten mit Variationen in der Expression der Gene rechnen muß, da diese von der chromosomalen Lokalisation beeinflußt wird (Schell 1987). Die Kontrollkonstrukte (ΔAntisense-PPDK und β-Glucoronidase) verhielten sich in allen Fällen wie der Wildtyp. Im weiteren werden sie daher nicht immer gesondert erwähnt.

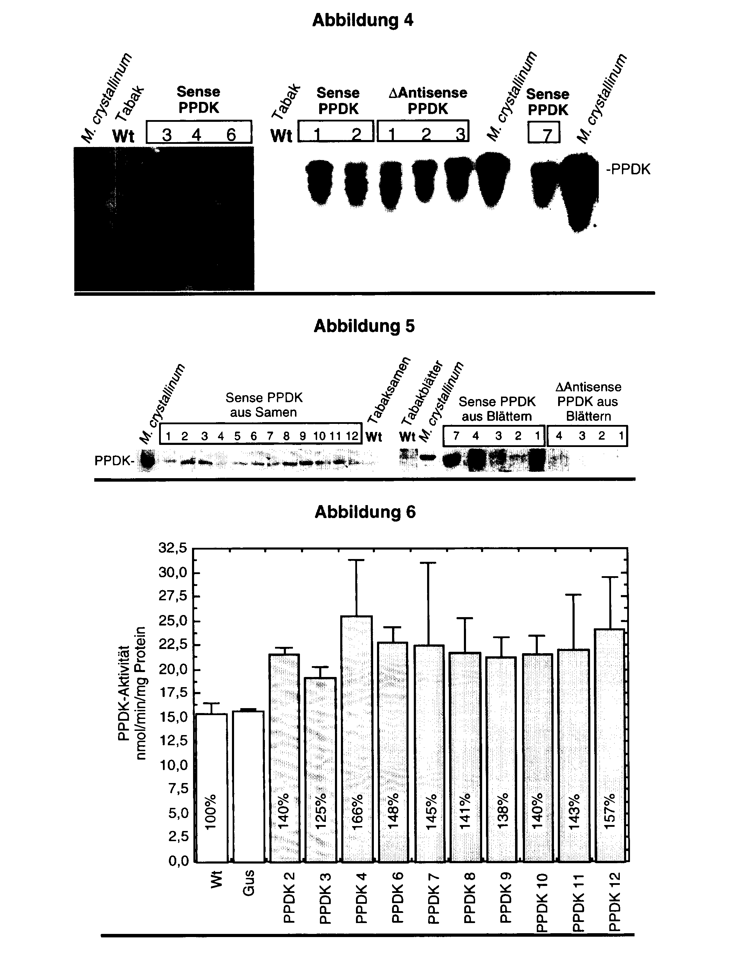

Die Expression der heterologen Gene in Tabakblättern ist in Abbildung 4 gezeigt. Sowohl Sense-, wie auch ΔAntisene-mRNAs wurden hierbei detektiert, da bei der Hybridisierung mit einer doppelsträngigen Sonde operiert wurde. Der mRNA-Spiegel der heterolog exprimierten PPDK war etwa 1/4x so hoch wie in Blättern von M. *crystallinum* im CAM-Zustand. Der Tabak-Wildtyp zeigte unter den gewählten Reaktionsbedingungen keinerlei *ppdk-mRNA*-Signal. Abbildung 5 zeigt, daß in den Sense-PPDK-Pflanzen die PPDK produziert wurde und in Samen wie auch in Blättern dieser Pflanzen zu finden war. Im Verhältnis zu salzgestreßten M. *crystallinum-Pflanzen* war die Expressionshöhe des Proteins allerdings sehr gering und steht damit im Kontrast zum mRNA-Spiegel, der bei den meisten Pflanzen recht hoch war. Im Wildtyp wie auch in den ΔAntisense-PPDK-Pflanzen konnten unter diesen Bedingungen keine oder nur geringe PPDK-Mengen detektiert werden.

Abbildung 6 zeigt, daß die PPDK-überexprimierenden Tabakpflanzen auch erhöhte PPDK-Aktivität (125 - 166% der Aktivität des Wildtyps) besaßen. Die Kontrolltransformante mit dem β-Glucuronidase-Gen (Gus) zeigte keine erhöhte PPDK-Aktivität. Die PPDK-Aktivität des Wildtyps entsprach den in der Literatur für Tabak beschriebenen Werten (Edwards *et al.* 1980; Kisaki *et al.* 1973).

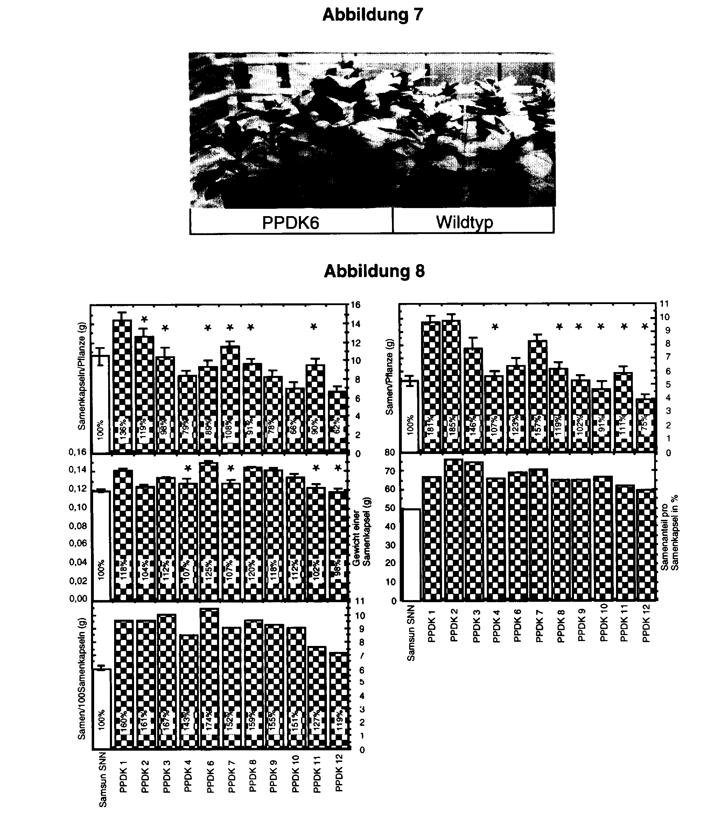

Als nächstes wurde die Produktivität der PPDK-überexprimierenden Tabaklinien ermittelt. Hierzu muß zunächst angemerkt werden, daß die PPDK-überexprimierenden Tabakpflanzen schneller wuchsen als der Wildtyp und ungefähr drei Wochen früher Samen produzierten (Abbildung 7).

Die in Abbildung 8 gezeigten Daten befassen sich allein mit dem Effekt der zusätzlichen PPDK auf die Samenproduktion. Auffälligerweise produzierten alle elf analysierten Linien mehr Samen pro Samenkapsel als der Wildtyp (= 100%). Den höchsten Wert erreichte hier die Linie PPDK 6 mit 174%. Diese Produktivitätssteigerung spiegelte sich auch im Verhältnis der Gewichtsanteile von Samen zu Samenkapsel wieder. Während die Samen beim Wildtyp gerade 50% des Gewichtes der Samenkapsel ausmachten, machten die Samen bei den PPDK-überexprimierenden Pflanzen um die 65% des Gewichts der Samenkapsel aus. Am effektivsten war hier die Linie PPDK 2 mit 78%. Zusätzlich war das Gewicht der Samenkapseln an sich in den meisten der PPDK-überexprimierenden Tabaklinien höher als beim Wildtyp. Den Spitzenwert erreichte hier wieder die Linie PPDK 6 mit 125%. Folgerichtig produzierten die meisten dieser Linien auch insgesamt mehr Samen als der Wildtyp. PPDK 2 erreichte hier mit 185% den höchsten Wert.

Zusammenfassend kann hier festgestellt werden, daß die PPDK-überexprimierenden Tabakpflanzen wahrscheinlich effizienter mit der Energie umgingen, die den Samenkapseln zur Verfügung stand. Sie produzierten nicht in jedem Falle mehr Samenkapseln oder Samen als der Wildtyp, aber auf jeden Fall mehr Samen pro Samenkapsel (bis zu 74% mehr). Zusätzlich wuchsen diese Pflanzen schneller als der Wildtyp.

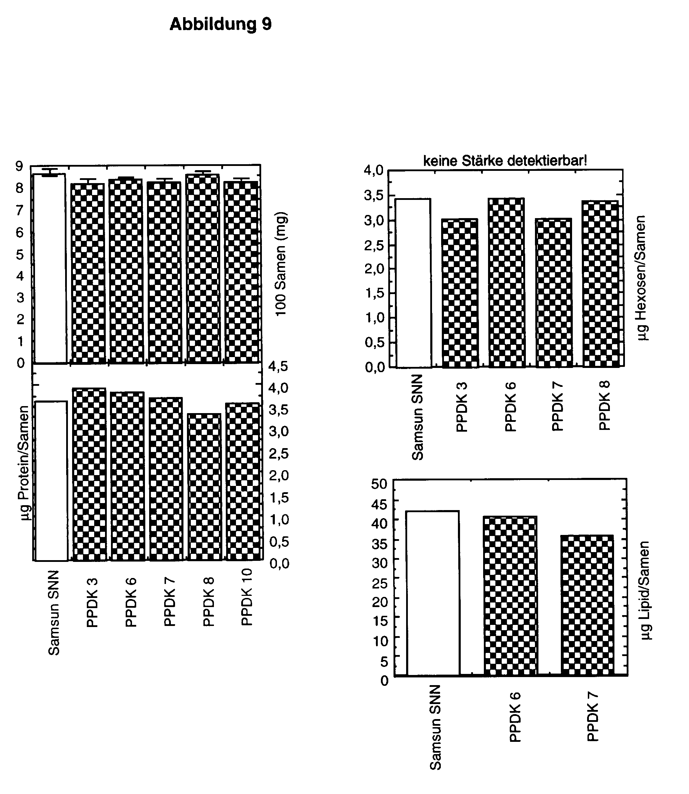

Die Samen, die der PPDK-überexprimierende Tabak produzierte, waren, wie die des Wildtyps, zu 90% keimungsfähig. Auch sonst waren keine signifikanten Veränderungen im Gewicht oder in der Zusammensetzung der Samen zu beobachten. Abbildung 9 zeigt, daß die Samen das gleiche Gewicht wie die Samen des Wildtyps aufwiesen und auch die gleichen Protein-, Lipid- und Zuckermengen akkumulierten. Dabei zeigte sich, daß die Tabaksamen keine Stärke, sondern lösliche Zucker speicherten. Die Hauptspeichersubstanzen in den kleinen Tabaksamen waren, wie erwartet, Lipide (ca. 48% des Samengewichts). Freie Zucker und Proteine machten nur etwa 8% des Samengewichtes aus. Die Eigenschaften der Tabaksamen wurden durch die erhöhte Effizienz der Energieverwertung in den beobachteten Kriterien nicht verändert.

### Schlußfolgerungen

Aus den Daten lassen sich folgende Schlüsse ziehen: Mit Hilfe zusätzlicher plastidärer PPDK können Pflanzen zu schnellerem Wachstum veranlaßt werden. Diese Pflanzen produzieren mehr Samen als der Wildtyp. Die erhöhte Samenproduktion kommt durch schwerere Samenkapseln und höhere Samenmengen pro Samenkapsel zustande. Der Grund für diese Effekte konnte nicht eindeutig geklärt werden, liegt aber mit großer Wahrscheinlichkeit an der Refixierung von Kohlendioxid (siehe Abbildung 1), welches während der Atmung entsteht, da auch nicht-photosynthetisch aktive Gewebe wie Samen vom zusätzlichen Phospho*enol*pyruvat profitieren. Dies führt zu effizienterer Nutzung der zur Verfügung stehenden Kohlenstoffgerüste. Die zusätzliche plastidäre PPDK stellt das für die Refixierung durch PEPCase benötigte Substrat Phosphoenolpyruvat in größeren Mengen zur Verfügung und behebt so einen zu Energieverschwendung und Kohlenstoffverlust führenden Engpaß. Auch die geringste gemessene Aktivitätserhöhung von 125% führt zu deutlich erhöhter Samenproduktionseffizienz und schnellerem Wachstum der Pflanzen (Abbildung 6, 7 und 8).

Durch die hier beschriebene gentechnische Veränderung mit einer plastidären PPDK sollte sich nahezu jede Pflanze in oben beschriebener Weise verbessern lassen. Die Gentechnik erlaubt zusätzlich, solche Veränderungen sehr schnell herbeizuführen, während die Züchtung eines solchen Merkmals zumindest ein sehr zeitaufwendiger Prozeß ist.

### Referenzen

**Aoyagi K. and Bassham J.A.** (1986) Appearance and accumulation of C₄ carbon pathway enzymes in developing wheat leaves. *Plant Physiol.* **80***,* 334-340.
**Bevan M.** (1984) Binary *Agrobacterium* vectors for plant transformation. *Nucl. Acids Res.* **12**, 8711-8721.
**Borlaug N.E. and Dowswell C.R.** (1988) World revolution in agriculture. In *Book of the Year 1988,* eds) Chicago: Encyclopedia Britannica, pp. pp. 514.
**Carroll N.V., Langley R.W. and Roe J.H.** (1956) The determination of glycogen in liver and muscle by use of anthrone reagent. J. *Biol. Chem.* **220**, 583-593.
**Dennis D.T. and Turpin D.H.** (1990) Plant Physiology, Biochemistry and Molecular Biology. Longman Group UK Limited, Essex
**Edwards G.E., Ujihira M. and Sugiyama T.** (1980) Light and temperature dependence of the rate and degree of activation of pyruvate, Pᵢ dikinase *in vivo* in maize. *Photosynth. Res.* **1**, 199-207.
**Fißlthaler B.** (1993) Die Pyruvat, Phosphat Dikinase aus der fakultativen Crassulaceen-Säurestoffwechsel-Pflanze *Mesembryanthemum crystallinum:* Sequenzierung und Charakterisierung des Gens. Freie Universität Berlin, Dissertation
**Fißlthaler B., Meyer G., Bohnert H.J. and Schmitt J.M.** (1995) Age-dependent induction of pyruvate, othophosphate dikinase in *Mesembryanthemum crystallinum* L. *Planta* **196**, 492-500.
**Horsch R.B., Fry J.E., Hoffmann N.L., Eichholtz D., Rogers S.G. and Fraley R.T.** (1985) A simple general method for transferring genes into plants. *Science* **227***,* 1229-1231.
**Kisaki T., Hirabayashi S. and Yano N.** (1973) Effect of the age of tobacco leaves on photosynthesis and photorespiration. *Plant Cell Physiol.* **14**, 505-514.
**Lambers H.** (1985) Respiration in intact plants and tissues. Its regulation and dependence on environmental factors, metabolism and invaded organisms. In *Higher Plant Cell Respiration,* (Douce R. and Day D.A., eds) Berlin: Springer-Verlag, pp. 418-473.
**Latzko E. and Kelly G.J.** (1983) The many-faceted function of phospho*enol*pyruvate carboxylase in C₃ plants. *Physiol. Veg.* **2**, 805-815.
**Meyer A.O., Kelly G.J. and Latzko E.** (1978) Pyruvate orthophosphate dikinase of immature wheat grains. *Plant Sci. Lett.* **12**, 35-40.
**Mohr H. and Schopfer P.** (1978) Lehrbuch der Pflanzenphysiologie. Springer-Verlag, Berlin
**Rosche E., Streubel M. and Westhoff P.** (1994) Primary structure of the photosynthetic pyruvate orthophosphate dikinase of the C₃ plant *Flaveria pringlei* and expression analysis of pyruvate orthophosphate dikinase sequences in C₃, C₃-C₄ and C₄ *Flaveria* species. *Plant Mol. Biol.* **26**, 763-769.
**Schell J.** (1987) Transgenic Plants as Tools to Study the Molecular Organization of Plant Genes. *Science* **237**, 1176-1183.
**Spector T.** (1978) Refinement of the Coomassie blue method of protein quantitation. *Anal. Biochem.* **86**, 142-146.
**Strasburger E., Noll F., Schenck H. and Schimper A.F.W.** (1991) Lehrbuch der Botanik. Gustav Fischer Verlag, Stuttgart
**Taiz L. and Zeiger E.** (1991) Plant Physiology. The Benjamin/Cummings Publishing Company, Inc., Redwood City
**Ting I.P.** (1985) Crassulacean acid metabolism. *Ann. Rev. Plant. Physiol.* **36**, 595-622. **Vancanneyt G., Schmidt R., O'Connor-Sanchez A., Willmitzer L. and Rocha-Sosa M.** (1990) Construction of an intron-containing marker gene: Splicing of the intron in transgenic plants and its use in monitoring early events in *Agrobacterium-mediated* plant transformation. MGG **220**, 245-250.
**Voet D. and Voet J.G.** (1992) Biochemie. VCH, Weinheim
**Wilson D. and Jones J.G.** (1982) Effect of selection for dark respiration rate of mature leaves on crop yields *of Lolium perenne* cv. S23. *Ann. Bot.* **49**, 313-320.
**Zöllner N. and Kirsch K.** (1962) Z. *ges. exp. Med.* **135**, 545.

### Legenden

**Abbildung 1**: Stoffwechselschema nach Latzko und Kelly (1983) zeigt eine Möglichkeit zur Refixierung respiratorischen Kohlendioxids.
**Abbildung 2:** Stoffwechselschema nach Aoyagi und Bassham (1986) zeigt eine Möglichkeit zur Kohlendioxidanreicherung.
**Abbildung 3:** Schematische Zeichnung der konstruierten binären Vektoren mit *M. crystallinum*-PPDK-Insertion. Ganz oben ist die PPDK-cDNA mit einigen Restriktionsschnittstellen dargestellt (Fißlthaler 1993). Die PPDK-cDNA-Sequenz aus *M. crystallinum* ist 3173 bp lang, von denen 9 bp am 5'-Ende die Linkersequenz aus der cDNA-Synthese und die 3'-terminalen 12 bp das nach der Transkription angehängte PolyA-Ende darstellen. Das Startkodon befindet sich an Position 63, das Terminationskodon des längsten offenen Leserasters an Position 2910. Das aus der cDNA-Sequenz der PPDK aus *M. crystallinum* abgeleitete Protein besteht aus 949 Aminosäuren, was einem Molekulargewicht von 103244 Dalton entspricht. Die Präsequenz der PPDK aus *M. crystallinum* hat eine Länge von 74 Aminosäuren, so daß das reife, im Chloroplasten prozessierte Protein, eine Größe von etwa 94 kDa hat. Die Aminosäurezusammensetzung des reifen, prozessierten Proteins ergibt einen pl von 7,6. 5'- und 3'-untranslatierte Bereiche der cDNA sind als terminale schraffierte Boxen dargestellt. In der Sense-PPDK ist die PPDK-cDNA in richtiger Orientierung dargestellt (links 5'-, rechts 3'-untranslatierter Bereich; die gepunktete Box stellt die Plastidensignalsequenz mit den drei im Leseraster befindlichen ATGs dar; das erste ATG wird normalerweise als Translationsstart benutzt). Δ zeigt eine Deletion von 183 bp am ursprünglichen 5'-Ende an.
   BR, BL: rechte bzw. linke Grenzregion der T-DNA von *Agrobacterium tumefaciens;* Km: Kanamycinresistenz mit bakteriellen Regulationselementen; nptll: Neomycinphosphotransferase (Kanamycinresistenz) mit pflanzlichen Regulationselementen; 35S CaMV: Promotor des Blumenkohlmosaikvirus; ocs polyA: Polyadenylierungssignal des Octopinsynthasegens von *Agrobacterium tumefaciens.*
   Die Transformation der Tabakpflanzen mit Hilfe des *A. tumefaciens*-vermittelten Gentransfers wurde nach Horsch *et al.* (1985) vollzogen.
**Abbildung 4:** Die *ppdk-mRNA* aus Blättern von PPDK-überexprimierenden Tabakpflanzen (Sense und ΔAntisense; siehe auch Abbildung 3) wurde durch Hybridisierung mit der M. *crystallinum-ppdk*-cDNA-Sonde auf einem Northern-Blot sichtbar gemacht. Blatt-RNA des untransformierten Tabaks (Wt) diente als Negativkontrolle und Blatt-RNA einer *M. crystallinum* im CAM-Zustand als Positivkontrolle. Die einzelnen PPDK-Mutanten sind in allen Abbildungen mit den selben Nummern versehen. Es wurden je 40 µg Tabak-RNA und 20 µg *M. crystallinum-*RNA eingesetzt.
**Abbildung 5:** PPDK-Proteinspiegel von PPDK-überexprimierenden Tabakpflanzen (siehe auch Abbildung 3) wurden durch Western-Blots untersucht. 2 µg Rohextrakt einer salzgestreßten *M. crystallinum* dienten als Positivkontrollen. Untransformierter Tabak (Wt) und AAntisense-Tabakpflanzen dienten als Negativkontrollen. 62 µg der löslichen Blattproteine und 45 µg der löslichen Samenproteine der Tabakpflanzen wurden eingesetzt. Bei den analysierten transgenen Pflanzen handelte es sich jeweils um die F1-Generation unabhängiger Transformanten.
**Abbildung 6:** PPDK-Aktivitätsmessungen nach Holtum und Winter (1982) aber ohne Pyruvat und KH2PO4 im Homogenisationsmedium. Die Bestimmung wurde aus Blattrohextrakten vorgenommen. Die Standardfehler von zehn (Gus, PPDK2 - 4 und PPDK7 - 12), 28 (PPDK6) und 41 (Wt) unabhängigen Messungen sind eingezeichnet. Der Tabak-Wildtyp (Wt; Samsun SNN), die Kontrolltransformanten mit dem β-Glucuronidase-Gen (Gus) und Tabaklinien mit zusätzlicher *Mesembryanthemum crystallinum*-PPDK wurden analysiert. Die Veränderungen der PPDK-Aktivität sind signifikant (t-test, P < 0.05, mit Ausnahme von PPDK8: P < 0.1).
**Abbildung 7:** Unterschiede in der Wachstumgeschwindigkeit von Wildtyp (rechts) und einer PPDK-überexprimierenden Tabaklinie (PPDK6) vier Monate nach Aussaat. Eine transgene Linie wurde als Beispiel ausgewählt.
**Abbildung 8:** Ertragsanalyse der PPDK-überexprimierenden Tabaklinien. Für die Analyse wurden 33 Wildtyp-Tabakpflanzen und 155 der PPDK-überexprimierenden Tabakpflanzen bis zur Samenreife herangezogen und ausgewertet. Die einzelnen unabhängigen Transformanten (F1-Generation) wurden in folgenden Mengen analysiert: Transformant Nr. 6: 16 Pflanzen; Transformant Nr. 1, 2, 8, 9, 12: je 15 Pflanzen; Transformant Nr. 4: 14 Pflanzen; Transformant Nr. 3, 10: je 13 Pflanzen; Transformant Nr. 7, 11: je 12 Pflanzen. Die Standardfehler sind eingezeichnet. Nicht-signifikante Veränderungen sind durch ein Sternchen gekennzeichnet (*; t-Test, P > 0.1). Alle anderen sind signifikant (t-Test, P < 0.05).
**Abbildung 9:** Zusammensetzung der Samen der PPDK-überexprimierenden Tabaklinien. Für die Bestimmung des Samengewichtes wurden 6 x 100 Wildtyp-Samen und 3 x 100 Samen von fünf PPDK-überexprimierenden Tabaklinien ausgewogen. Die Proteinmengen der Wildtyp-Samen und aller Transformanten-Samen (je 100 mg) wurden mit Coomassie Blau bestimmt (Spector 1978). Zu sehen sind nur die Werte für Samen, deren Gewicht auch bestimmt wurde. Alle anderen Transformanten-Samen akkumulierten im Rahmen der Meßgenauigkeit die gleichen Proteinmengen. Die Zuckeranalyse wurde nach Carroll *et al.* (1956) durchgeführt. 100 mg Samen wurden homogenisiert und mit 2 ml 25% Ethanol bei 70°C für 30 min inkubiert. Die Messungen wurden mit Glucose standardisiert.

Die Lipidanalyse wurde mit 100 mg Samen von zwei Transformanten und zwei Wildtypproben nach Zöllner und Kirsch (1962) durchgeführt. Die Messungen wurden mit Sonnenblumenöl standardisiert. Veränderungen sind nicht signifikant (t-Test, P < 0.05).

## Patentansprüche

1. Verfahren zur Erhöhung der pflanzlichen Samenproduktion und zur Beschleunigung des Pflanzenwachstums, dadurch gekennzeichnet, daß ein zusätzliches Gen für eine in den Plastiden lokalisierte Pyruvat, Phosphat Dikinase (PPDK; EC 2.7.9.1) oder ein anderes Enzym bzw. eine Enzymabfolge, welche Pyruvat in Phosph*enol*pyruvat umwandeln kann, in Pflanzenzellen integriert wird und dadurch in den Plastiden von Pflanzen zusätzliches Phosph*enol*pyruvat produziert wird.

2. Verfahren nach Anspruch 1, dadurch gekennzeichnet, daß beispielsweise die PPDK-cDNA aus der fakultativen CAM-Pflanze *Mesembryanthemum crystallinum* in Pflanzen eingebracht und überexprimiert wird.
